# EUROPEAN PATENT SPECIFICATION

(11) **EP 0 706 377 B1**
(45) Date of publication and mention of the grant of the patent: **05.12.2001**
(21) Application number: 95914917.0
(22) Date of filing: 21.03.1995
(51) Int. Cl.: A61K 9/22, A61K 9/20

(54) **SUSTAINED RELEASE EXCIPIENT**
TRÄGERSTOFF ZUR VERZÖGERTEN FREISETZUNG
EXCIPIENT A LIBERATION PROLONGEE

(30) Priority: 25.04.1994 US 232625
(43) Date of publication of application: 17.04.1996
(60) Divisional application: 01112778.4
(73) Proprietor: EDWARD MENDELL CO., INC., Patterson, NY 12563-9970 (US)
(72) Inventor: BAICHWAL, Anand R., Wappingers Falls, NY 12590 (US); McCALL, Troy W., New Milford, CT 06776 (US)
(74) Representative: Woodward, John Calvin
(86) International application number: US9503825
(87) International publication number: WO9528916

(56) References cited:
- EP-A- 0 265 116
- EP-A- 0 311 582
- EP-A- 0 341 745
- EP-A- 0 360 562
- EP-A- 0 642 785
- WO-A-95/13055
- GB-A- 2 264 866
- US-A- 4 303 691
- US-A- 4 309 405
- US-A- 4 764 380
- US-A- 4 795 642
- US-A- 5 128 143
- US-A- 5 169 639

## Description

### FIELD OF THE INVENTION

The present invention relates to sustained release excipient formulations which may be blended with a wide range of therapeutically active medicaments and made into sustained release oral solid dosage forms.

### BACKGROUND OF THE INVENTION

In our U.S. Patent Nos. 4,994,276; 5,128,143; and 5,135,757, hereby incorporated by reference, we reported that a controlled release excipient which is comprised of synergistic heterodisperse polysaccharides (e.g., a heteropolysaccharide such as xanthan gum in combination with a polysaccharide gum capable of cross-linking with the heteropolysaccharide, such as locust bean gum) is capable of processing into oral solid dosage forms using either direct compression, following addition of drug and lubricant powder, conventional wet granulation, or a combination of the two. The release of the medicament from the formulations therein proceeded according to zero-order or first-order mechanisms.

The controlled release excipients disclosed in U.S. Patent Nos. 4,994,276, 5,128,143, and 5,135,757 are commercially available under the tradename TIMERx™ from Edward Mendell Co., Inc., Patterson, N.Y., which is the assignee of the present invention.

European Pat. No. 234670 B (Pankhania et al.) describes a sustained-release pharmaceutical formulation containing xanthan gum wherein the xanthan gum comprises from about 7.5 to about 28 percent, by weight, of the formulation except for a formulation wherein the sustained release carrier comprises a mixture of 15-50 parts by weight dimethylsiloxane, 30-100 parts by weight silicic acid, 30-100 parts by weight mannans or galactans or a mixture thereof, 50-150 parts by weight xanthans and 5-75 parts by weight micronized seaweed.

### OBJECTS AND SUMMARY OF THE INVENTION

It is an object of the present invention to provide new sustained release matrices which, when incorporated into a final product, may cause the release of therapeutically active medicaments over an extended period of time when the dosage form is exposed to fluids in an environment of use, e.g. from about 12 to about 24 hours or more.

In accordance with the above-mentioned object, and others which will be apparent from the following disclosure, the present invention is related to sustained-release excipient for use in oral solid dosage forms, comprising from 15 to 30 percent or more by weight heteropolysaccharide gum; an effective amount of a cationic cross-linking agent capable of cross-linking the heteropolysaccharide in an environment of use; and an inert pharmaceutical filler. In certain preferred embodiments, the cationic cross-linking agent comprises from 1 to 20 percent by weight of the sustained-release matrix. In additional preferred embodiments, the inert pharmaceutical filler comprises from 60 to 85 percent by weight of the sustained-release matrix.

The sustained-release matrices of the present invention can be mixed with a wide range of therapeutically active medicaments and thereafter compressed into solid dosage forms such as tablets. The solid dosage forms thus made slowly release the medicament over about a 24-hour time period when ingested and exposed to an environment of use, e.g. gastric fluids. By varying the amount of excipient relative to the medicament, a desired sustained-release profile can be attained.

In preferred embodiments the heteropolysaccharide comprises xanthan gum.

In another preferred embodiment the cationic cross-linking agent is calcium sulfate.

The present invention also provides a 24-hour sustained-release tablet for oral administration comprising (I) a hydrophilic material comprising (a) a heteropolysaccharide; or (b) a heteropolysaccharide and a cationic cross-linking agent capable of cross-linking said heteropolysaccharide; and (II) an inert pharmaceutical filler comprising from 60 to 85 percent by weight of the hydrophilic material; and (III) an effective amount of a therapeutically active ingredient.

In addition, the present invention provides a method for providing a sustained release matrix for sustained release dosage forms containing one or more therapeutically active medicaments, comprising preparing a sustained-release matrix by dry blending the requisite amounts of heteropolysaccharide gum, inert pharmaceutical filler, and cationic cross-linking agent. In certain preferred embodiments the sustained release excipient is prepared by dry blending the requisite amounts of heteropolysaccharide gum, inert pharmaceutical filler, and cationic cross-linking agent, wet granulating the mixture, and then drying the mixture to obtain the final sustained release excipient. The sustained release excipient thereby obtained may then be directly admixed with an active ingredient along with any further pharmaceutically necessary inert excipients, and then formulated into a final oral solid dosage form.

After admixture with the therapeutically active medicaments, the sustained release excipient/drug mixture may then be manufactured into a final dosage form, e.g., by directly compressing the mixture into tablets.

### DETAILED DESCRIPTION OF THE INVENTION

The excipients of the present invention have been pre-optimized by providing a sustained-release excipient product which may be mixed with a wide range of medicaments and made into oral solid dosage forms capable of releasing the active medicament in the environment of use over about a 12 to 24 hour time period, without the aid of the usual pharmaceutical dry or wet binders, fillers, disintegrants, glidants etc., which must be added in many prior art compositions to obtain an acceptable solid dosage form. Thus, the excipients of the present invention substantially overcome the need for conducting further experimentation needed to optimize release characteristics and tabletting properties for a particular therapeutically active medicament.

In other words, the controlled release excipient used in the present invention provides a product which contains a combination of ingredients in preselected proportions to each other which provides a desired controlled release profile over a 12 to at least a 24-hour period for a wide variety of drugs. Thus, once the excipient product is admixed with an active medicament (and preferably with a lubricant) in a ratio to the sustained-release excipient in accordance with the present invention, the resulting mixture may be made into oral solid dosage forms capable of releasing an active medicament over an extended period of time.

Xanthan gum, the preferred heteropolysaccharide, is produced by microorganisms, for instance, by fermentation with the organism xanthomonas compestris. Most preferred is xanthan gum which is a high molecular weight (>10⁶) heteropolysaccharide. Xanthan gum contains D-glucose, D-mannose, D-glucuronate in the molar ratio of 2.8:2.0:20, and is partially acetylated with about 4.7% acetyl. Xanthan gum also includes about 3% pyruvate, which is attached to a single unit D-glucopyromosyl side chain as a metal. It dissolves in hot or cold water and the viscosity of aqueous solutions of xanthan gum is only slightly affected by changes in the pH of a solution between 1 and 11.

The term "heteropolysaccharide" as used in the present invention is defined as a water-soluble polysaccharide containing two or more kinds of sugar units, the heteropolysaccharide having a branched or helical configuration, and having excellent water-wicking properties and immense thickening properties. When admixed with an appropriate cationic cross-linking agent capable of cross-linking with the heteropolysaccharide in accordance with the present invention and exposed to an aqueous solution, gastric fluid, etc., the gum packs closely and many intermolecular attachments are formed which make the structure strong and provide a hydrophilic gum matrix having high gel strength. The cationic cross-linking agent is therefore an agent capable of cross-linking the heteropolysaccharide, thus affecting the rate of release of the active medicament.

The cationic cross-linking agent may be monovalent or multivalent metal cations. The preferred salts are the inorganic salts, including various alkali metal and/or alkaline earth metal sulfates, chlorides, borates, bromides, citrates, acetates, lactates, etc. Specific examples of suitable cationic cross-linking agents include calcium sulfate, sodium chloride, potassium sulfate, sodium carbonate, lithium chloride, tripotassium phosphate, sodium borate, potassium bromide, potassium fluoride, sodium bicarbonate, calcium chloride, magnesium chloride, sodium citrate, sodium acetate, calcium lactate, magnesium sulfate and sodium fluoride or mixtures thereof. Multivalent metal cations may also be utilized. However, the preferred cationic cross-linking agents are bivalent. Particularly preferred salts are calcium sulfate and sodium chloride. The cationic cross-linking agents of the present invention are added in an amount effective to obtain a desirable increased gel strength due to the cross-linking of the gelling agent (e.g., the heteropolysaccharide gums).

Two steps which are generally required for gelation are the fast hydration of the macromolecules which comprise the hydrophilic material and thereafter the association of the molecules to form gels. Thus, two important properties of a hydrophilic gel matrix which are needed for application in a sustained-release system are the fast hydration of the system and a matrix having a high gel strength. As noted above, the cationic cross-linking agent may affect the hydration process of the heteropolysaccharide. These two important properties which are needed for application in a sustained-release system are the fast hydration of the system and a matrix having a high gel strength. These two important properties which are necessary to achieve a slow release hydrophilic matrix are maximized in the present invention by the particular combination of materials. In particular, heteropolysaccharides such as xanthan gum have excellent water wicking properties which provide fast hydration. On the other hand, the combination of xanthan gum with cationic cross-linking materials and the like which are capable of cross-linking the rigid helical ordered structure of the xanthan gum and can alter the gelation process and thus effect the rate of release of the active medicament.

In the present invention, it has been discovered that the controlled release properties of the tablets are optimized when the ratio of xanthan gum to cationic cross-linking agent (e.g., calcium sulfate, etc.) is from 1:1 to 3.5:1 and most preferably from 1.5:1 to 3:1, although xanthan gum in an amount of from 15 to 30 percent or more by weight of the sustained release excipient provides an acceptable slow release product.

In one preferred embodiment, the cationic cross-linking agent comprises calcium sulfate, and is present in the sustained release excipient in an amount of about 10 percent, by weight of the excipient. The ratio of the heteropolysaccharide to the cationic cross-linking agent is preferably from 1.5:1 to 3:1.

Any generally accepted soluble or insoluble inert pharmaceutical filler (diluent) material can be used. Preferably, the inert pharmaceutical filler comprises a monosaccharide, a disaccharide, a polyhydric alcohol, a cellulose (such as microcrystalline cellulose) and/or mixtures thereof. Examples of suitable inert pharmaceutical fillers include sucrose, dextrose, lactose, microcrystalline cellulose, xylitol, fructose, sorbitol, starches, and mixtures thereof. However, it is preferred that a soluble pharmaceutical filler such as dextrose, sucrose, or mixtures thereof be used.

In certain preferred embodiments of the invention, the sustained release matrix further comprises a hydrophobic material in an amount effective to slow the hydration of the gum without disrupting the hydrophilic matrix formed by the heteropolysaccharide when the formulation is exposed to fluids in an environment of use. This may be accomplished by granulating the sustained release matrix with a solution or dispersion of hydrophobic material prior to the incorporation of the medicament. The hydrophobic material may be selected from ethylcellulose, acrylic and/or methacrylic acid polymers or copolymers, hydrogenated vegetable oils, zein, as well as other pharmaceutically acceptable hydrophobic materials known to those skilled in the art. Other hydrophobic cellulosic materials such as other alkyl celluloses may also be used. The amount of hydrophobic material incorporated into the sustained release matrix is that which is effective to slow the hydration of the gums without disrupting the hydrophilic matrix formed upon exposure to an environmental fluid. In certain preferred embodiments of the present invention, the hydrophobic material may be included in the sustained release matrix in an amount from 1% to 20% by weight. More preferably, the hydrophobic material may be included in the sustained release matrix in an amount from 3% to 12%, and most preferably from 5% to 10%, by weight of the final formulation. The hydrophobic material may be dissolved in an organic solvent or dispersed in an aqueous solution for incorporation into the formulation.

The combination of the hydrophilic material (e.g., xanthan gum) with the cationic cross-linking agent and inert diluent provides a ready to use sustained-release excipient in which a formulator need only blend the desired active medicament and an optional lubricant with the excipient and then make an oral solid dosage form. The sustained-release excipient may thus comprise a physical admix of the heteropolysaccharide along with a cationic cross-linking agent, or soluble excipient such as sucrose, lactose or dextrose.

One of the limitations of direct compression as a method of tablet manufacture is the size of the tablet. For example, where the dosage form is an oral sustained release tablet and the dose of therapeutically active agent to be contained in the tablet is relatively large, a pharmaceutical formulator may choose to wet granulate the drug with other excipients to attain a desired tablet size with the correct compact strength (e.g., hardness). Usually the amount of filler/binder or excipients needed in wet granulation is less than that in direct compression since the process of wet granulation contributes to some extent toward the desired physical properties of a tablet. Accordingly, the sustained release pharmaceutical excipient prepared in accordance with the present invention may be subjected to wet granulation before the medicament is added. In this technique, the desired amounts of the heteropolysaccharide, the cationic cross-linking agent, and the inert filler are mixed together and thereafter a moistening agent such as water, propylene glycol, glycerol, alcohol or the like is added to prepare a moistened mass. Next, the moistened mass is dried. The dried mass is then milled with conventional equipment into granules. Therefore, the sustained-release excipient product is ready to use. The granulate thus obtained has certain advantages including the fact that it is free-flowing, has good cohesive properties, and can be admixed with an active agent (e.g., drug) and can be directly compressed into tablets. On the other hand, the granulate can be formulated into a capsule, used in the granulate form, extruded, and/or spheronized with an active medicament to form pellets, etc.

Alternatively, it is possible to dry mix the ingredients of the sustained release excipient without utilizing a wet granulation step. This procedure may be utilized, for example, where a wet granulation step is to be accomplished when the active ingredient is directly added to the ingredients of the sustained release excipient. On the other hand, this procedure may also be used where no wet granulation step whatsoever is contemplated. If the mixture is to be manufactured without a wet granulation step, and the final mixture is to be tabletted, it is preferred that all or part of the inert diluent comprise a pre-manufactured direct compression diluent. Such direct compression diluents are widely used in the pharmaceutical arts, and may be obtained from a wide variety of commercial sources. Examples of such pre-manufactured direct compression excipients include Emcocel® (microcrystalline cellulose, N.F.), Emdex® (dextrates, N.F.), and Tab-Fine® (a number of direct-compression sugars including sucrose, fructose, and dextrose), all of which are commercially available from Edward Mendell Co., Inc., Patterson, New York). Other direct compression diluents include Anhydrous lactose (Lactose N.F., anhydrous direct tabletting) from Sheffield Chemical, Union, N.J. 07083; Elcems® G-250 (Powdered cellulose, N.F.) from Degussa, D-600 Frankfurt (Main) Germany; Fast-Flo Lactose® (Lactose, N.F., spray dried) from Foremost Whey Products, Banaboo, WI 53913; Maltrin® (Agglomerated maltrodextrin) from Grain Processing Corp., Muscatine, IA 52761; Neosorb 60® (Sorbitol, N.F., direct-compression) from Roquette Corp., 645 5th Ave., New York, NY 10022; Nu-Tab® (Compressible sugar, N.F.) from Ingredient Technology, Inc., Pennsauken, NJ 08110; Poly-plasdone XL® (Crospovidone, N.F., cross-linked polyvinylpyrrolidone) from GAF Corp., New York, NY 10020; Primojel® (Sodium starch glycolate, N.F., carboxymethyl starch) from Generichem Corp., Little Falls, NJ 07424; Solka Floc® (Cellulose floc) from Edward Mendell Co., Carmel, NY 10512; Spray-dried lactose® (Lactose N.F., spray dried) from Foremost Whey Products, Baraboo, WI 53913 and DMV Corp., Vehgel, Holland; and Sta-Rx 1500® (Starch 1500) (Pregelatinized starch, N.F., compressible) from Colorcon, Inc., West Point, PA 19486.

In general, the formulator may prepare a directly compressible diluent, by wet granulating or spray drying lactose, for example. For purposes of the present invention, these specially treated inert diluents will be referred to as "directly compressible" inert diluents.

In further embodiments of the present invention, the directly compressible inert diluent which is used in conjunction with the sustained release pharmaceutical excipient of the present invention is an augmented microcrystalline cellulose as disclosed in U.S. Patent Application Serial No. 08/370,576, filed January 9, 1995, and entitled "PHARMACEUTICAL EXCIPIENT HAVING IMPROVED COMPRESSIBLITY", inventors J. Staniforth, B. Sherwood and E. Hunter.

Once the sustained release excipient of the present invention has been prepared, it is then possible to blend the same with an active medicament, metoprolol, e.g., in a V-blender. The mixture may then be manufactured into the desired final dosage form. If desired, the mixture can be directly compressed into tablets, or subjected to other intermediate processing steps such as wet granulation.

The dosage forms of the present invention are preferably tablets. However, the ingredients may also be formulated in a capsule, extruded and spheronized with an active medicament to form pellets, etc.

For example, the complete mixture, in an amount sufficient to make a uniform batch of tablets, is then subjected to tabletting in a conventional production scale tabletting machine at normal compression pressure, i.e. about 1.379 x 10⁷ to 1.103 x 10⁷ Pa (2000-1600 lbs/sq in). However, the mixture should not be compressed to such a degree that there is subsequent difficulty in its hydration when exposed to gastric fluid. An effective amount of any generally accepted pharmaceutical lubricant, including the calcium or magnesium soaps may be added to the above-mentioned ingredients of the excipient be added at the time the medicament is added, or in any event prior to compression into a said dosage form. One preferred lubricant is Pruv® , e.g., in the amount of about 3.0 percent of the solid dosage form.

The average tablet size for round tablets is preferably 500 mg to 750 mg and for capsule-shaped tablets 750 mg to 1000 mg.

The average particle size of the granulated excipient of the present invention ranges from 50 microns to 400 microns and preferably from 185 microns to 265 microns. The particle size of the granulation is not narrowly critical, the important parameter being that the average particle size of the granules, must permit the formation of a directly compressible excipient which forms pharmaceutically acceptable tablets. The desired tap and bulk densities of the granulation of the present invention are normally between from 0.3 to 0.8 g/ml, with an average density of from 0.5 to 0.7 g/ml. For best results, the tablets formed from the granulations of the present invention are from 6 to 8 kg hardness. The average flow of the granulations prepared in accordance with the present invention are from 25 to 40 g/sec.

Variables which may affect the release rate and the compressibility of tablets prepared with the excipient of the present invention are the drug to gum ratio; the method of incorporation of excipient (method of granulation); the relative amount of the gum to cationic cross-linking agent; and the ratio of active medicament to the sustained-release excipient.

The sustained release excipient formulations of the present invention may be utilized in the preparation of a wide range of 24 hour solid dosage forms which include a wide range of water-soluble or water-insoluble medicaments. Examples of such therapeutically active agents include antihistamines (e.g., dimenhydrinate, diphenhydramine, chlorpheniramine and dexchlorpheniramine maleate), analgesics (e.g., aspirin, codeine, morphine, dihydromorphone, oxycodone, etc.), anti-inflammatory agents (e.g., naproxen, diclofenac, indomethacin, ibuprofen, aspirin, sulindac), acetaminophen, gastro-intestinals and anti-emetics (e.g., metoclopramide), anti-epileptics (e.g., phenytoin, meprobamate and nitrezepam), vasodilators (e.g., nifedipine, papaverine, diltiazem and nicardipine), anti-tussive agents and expectorants (e.g., codeine phosphate), anti-asthmatics (e.g. theophylline), anti-spasmodics (e.g., atropine, scopolamine), hormones (e.g., insulin, heparin), diuretics (e.g., ethacrynic acid, bendroflumethiazide), anti-hypotensives (e.g., propranolol, clonidine), bronchodilators (e.g., albuterol), anti-inflammatory steroids (e.g., hydrocortisone, triamcinolone, prednisone), antibiotics (e.g., tetracycline), antihemorrhoidals, hypnotics, psychotropics, antidiarrheals, mucolytics, sedatives, decongestants, laxatives, antacids, vitamins, stimulants (including appetite suppressants such as phenylpropanolamine). The above list is not meant to be exclusive.

Upon oral ingestion and contact with gastric fluid, the controlled release tablets prepared according to the present invention swell and gel to form a hydrophilic gel matrix from which the drug is released. The swelling of the matrix causes a reduction in the bulk density of the tablet and provides the buoyancy necessary to allow the gel mass to float on the stomach contents to provide a slow delivery of the medicament. The matrix, the size of which is dependent upon the size of the original tablet, can swell considerably and become obstructed near the opening to the pylorus. Since the medicament is dispersed throughout the tablet (and consequently throughout the gel matrix), a constant amount of drug can be released per unit time in vivo by dispersion or erosion of the outer portions of the matrix. This phenomenon is commonly referred to as a zero order release profile or zero order kinetics. The process continues, with the matrix remaining buoyant in the stomach, until substantially all of the medicament is released. The chemistry of certain of the ingredients comprising the excipients of the present invention such as xanthan gum is such that the excipients are considered to be self-buffering agents which are substantially insensitive to the solubility of the medicament and likewise insensitive to the pH changes along the length of the gastrointestinal tract. Moreover, the chemistry of the ingredients comprising the excipients of the present invention is believed to be similar to certain known mucoadhesive substances such as polycarbophil. Mucoadhesive properties are desirable for buccal delivery systems. Thus, it may be possible that the gel system could potentially loosely interact with the mucin in the gastrointestinal tract and thereby provide another mode by which a constant rate of delivery of the medicament is achieved. The above hypothesis is included for discussion purposes only and is not intended to limit the scope of the present invention.

These two phenomenons, i.e., buoyancy of the gel matrix and the mucoadhesive properties discussed above, are possible mechanisms by which the gel matrix of the present invention could interact with the mucin and fluids of the gastrointestinal tract and provide a constant rate of delivery of the medicament. Other mechanisms are possible and therefore this hypothesis is not meant to limit the scope of the present invention.

### DETAILED DESCRIPTION OF THE PREFERRED EMBODIMENTS

The following examples illustrate various aspects of the present invention. They are not to be construed to limit the claims in any manner whatsoever.

### EXAMPLES 1-3

The sustained release excipient is prepared by dry blending the requisite amounts of xanthan gum, dextrose and calcium sulfate in a high-speed mixer/granulator for 2 minutes. While running choppers/impellers, the water is added and the mixture is granulated for another 2 minutes. The granulation is then dried in a fluid bed dryer to a loss on drying weight (LOD) of between 4 and 7%. The granulation is then milled using 20 mesh screens. The ingredients of the sustained release excipient of Example 1 are set forth in Table 1 below:

**TABLE 1**

| **PREPARATION OF SUSTAINED RELEASE EXCIPIENT** | | | |
|---|---|---|---|
| Component | %-Ex. 1 | %-Ex. 2 | %-Ex. 3 |
| 1. Xanthan gum | 30 | 15 | 30 |
| 2. Dextrose | 60 | 75 | 70 |
| 3. Calcium Sulfate | 10 | 10 | 0 |
| 4. Water | 10* | 10* | 10* |

| | | | |
|---|---|---|---|
| *removed during processing | | | |

Next, the sustained release excipient prepared a detailed above is dry blended with a desired amount of medicament (in the following examples metoprolol, provided, as the tartrate salt) in a V-blender for 10 minutes. A suitable amount of tabletting lubricant Pruv® (sodium stearyl fumarate, NF, commercially available from the Edward Mendell Co., Inc.) for the following examples is added and the mixture is blended for another 5 minutes. This final mixture is compressed into tablets, each tablet containing 100 mg metoprolol. The tablets of Example 1 weighed 618.5 mg. The tablets of Example 2 weighed 618.5 mg. The tablets of Example 3 weighed 618.5 mg. The drug:gum ratio of the tablets of Example 1 was 1:1.5. The drug:gum ratio of the tablets of Example 2 was 1:0.75. The drug:gum ratio of the tablets of Example 3 was 1:1.5. The ingredients of the tablets of Examples 1-3 are set forth in Table 2 below:

**TABLE 2**

| Component | % |
|---|---|
| 1. Sustained Release Excipient | 80.8% |
| 2. Metoprolol | 16.2% |
| 3. Pruv® | 3.0% |

Dissolution tests were then carried out on the tablets of Examples 1-3. The dissolution tests are conducted in an automated USP dissolution apparatus (Paddle Type II, pH 6.8 buffer, 100 rpm.). The results are set forth in Table 3 below:

**TABLE 3**

| **Effect of Single Gum Composition** | | | |
|---|---|---|---|
| Time(hours) | Example 1 | Example 2 | Example 3 |
| 0 | 0.0 | 0.0 | 0.0 |
| 2 | 25.3 | 29.0 | 20.7 |
| 4 | 37.9 | 42.7 | 32.3 |
| 8 | 56.3 | 63.6 | 50.2 |
| 12 | 70.6 | 77.9 | 64.1 |
| 16 | 81.3 | 88.2 | 74.3 |
| 20 | 89.0 | 94.9 | 81.3 |
| 24 | 97.6 | 98.8 | ― |

From the results provided in Table 3, it can be seen that formulations made with a greater concentration of gum produced slower drug release rates. It is also evident that the incorporation of calcium sulfate into single gum systems results in a faster drug release rates compared to formulations without calcium sulfate. Accordingly, the results provide that the tablets in Example 1 are suitable for delivering medicaments as an oral solid dosage form over a 24-hour oral period of time.

## Claims

1. A sustained release pharmaceutical excipient for oral solid dosage forms, comprising:
a heteropolysaccharide gum, the percentage of said heteropolysaccharide gum from 10 to 40 percent of the sustained release excipient,
a cationic cross-linking agent capable of cross-linking said heteropolysaccharide gum in the presence of aqueous solutions, the ratio of said heteropolysaccharide gum to said cationic cross-linking agent being from 1:1 to 3.5:1;
an inert pharmaceutical diluent selected from the group consisting of a monosaccharide, a disaccharide, a polyhydric alcohol, a cellulose, a starch, and mixtures thereof, the percentage of said inert diluent being from 60 to 85 percent by weight, of the sustained release excipient, and the sustained released excipient being in the form of a dry blend or a granulate.
wherein the sustained release pharmaceutical excipient is free from any homopolysaccharide gum.

2. The sustained release excipient of claim 1, wherein said inert pharmaceutical diluent is selected from the group consisting of lactose, dextrose, sucrose, fructose, microcrystalline cellulose, xylitol, sorbitol and mixtures thereof.

3. The sustained release excipient of claim 1, wherein the ratio of said inert pharmaceutical diluent to said heteropolysaccharide gum is from 6:1 to 2:1.

4. The sustained release excipient of claim 1, wherein said cationic cross-linking agent comprises from 1 to 20 percent of said sustained release excipient, by weight.

5. The sustained release excipient of claim 1, wherein said cationic cross-linking agent is selected from the group consisting of calcium sulfate, sodium chloride, potassium sulfate, sodium carbonate, lithium chloride, tripotassium phosphate, sodium borate, potassium bromide, potassium fluoride, sodium bicarbonate, calcium chloride, magnesium chloride, sodium citrate, sodium acetate, calcium lactate, magnesium sulfate, sodium fluoride and mixtures thereof.

6. The sustained release excipient of claim 1, wherein said cationic cross-linking agent is calcium sulfate.

7. The sustained release excipient of claim 1, wherein said heteropolysaccharide gum is xanthan gum.

8. The sustained release excipient of claim 1, wherein said excipient is in the form of a granulate.

9. The sustained release excipient of claim 1, wherein said inert pharmaceutical diluent is in a directly compressible form.

10. The sustained release excipient of claim 1, wherein said heteropolysaccharide gum, said inert pharmaceutical diluent, and said cationic cross-linking agent are granulated with a hydrophobic material selected from the group consisting of an alkylcellulose, a copolymer of acrylic and methacrylic acid esters, waxes, shellac, zein, hydrogenated vegetable oils, and mixtures of any of the foregoing, said hydrophobic material being included in an amount effective to slow the hydration of said gelling agent when exposed to an environmental fluid.

11. The sustained release excipient of claim 10, wherein said hydrophobic material is ethylcellulose.

12. A granulate useful as a sustained release pharmaceutical excipient for oral solid dosage forms, comprising:
a heteropolysaccharide gum, the percentage of said heteropolysaccharide gum from 10 to 40 percent of the sustained release excipient,
a cationic cross-linking agent capable of cross-linking said heteropolysaccharide gum in the presence of aqueous solutions, the ratio of said heteropolysaccharide gum to said cationic cross-linking agent being from 1:1 to 3.5:1;
an inert pharmaceutical diluent selected from the group consisting of a monosaccharide, a disaccharide, a polyhydric alcohol, a cellulose, a starch, and mixtures thereof, the percentage of said inert diluent being from 60 to 85 percent by weight, of the sustained release excipient;
said heteropolysaccharide gum, cationic cross-linking agent, and said inert pharmaceutical diluent having been agglomerated into granular particles via a wet granulation method.
wherein the sustained release pharmaceutical excipient is free from any homopolysaccharide gum.

13. The granulate of claim 12, wherein said inert pharmaceutical diluent is selected from the group consisting of lactose, dextrose, sucrose, fructose, microcrystalline cellulose, xylitol, sorbitol and mixtures thereof.

14. The granulate of claim 12, wherein the ratio of said inert pharmaceutical diluent to said heteropolysaccharide gum is from 6:1 to 2:1.

15. The granulate of claim 12, wherein said cationic cross-linking agent comprises from 1 to 20 percent of said sustained release excipient, by weight.

16. The granulate of claim 12, wherein said cationic cross-linking agent is selected from the group consisting of calcium sulfate, sodium chloride, potassium sulfate, sodium carbonate, lithium chloride, tripotassium phosphate, sodium borate, potassium bromide, potassium fluoride, sodium bicarbonate, calcium chloride, magnesium chloride, sodium citrate, sodium acetate, calcium lactate, magnesium sulfate, sodium fluoride and mixtures thereof.

17. The granulate of claim 12, wherein said cationic cross-linking agent is calcium sulfate.

18. The granulate of claim 12, wherein said heteropolysaccharide gum is xanthan gum.

19. A method for preparing a sustained release excipient of any one of claims 1-11, comprising:
wet granulating a heteropolysaccharide gum, a cationic cross-linking agent capable of cross-linking said heteropolysaccharide gum in the presence of aqueous solutions, and an inert pharmaceutical diluent selected from the group consisting of a monosaccharide, a disaccharide, a polyhydric alcohol, a cellulose, a starch, and mixtures thereof, and
drying the mixture to obtain distinct excipient particles.
wherein the sustained release excipient is free from any homopolysaccharide gum.

20. The method of claim 19, wherein the percentage of said heteropolysaccharide gum is from 10 to 40 percent of the granulate, the percentage of said inert diluent is from 60 to 85 percent by weight of the granulation, and the ratio of said heteropolysaccharide gum to said cationic cross-linking agent is from 1:1 to 3.5:1.

21. The method of claim 20, wherein said heteropolysaccharide gum is xanthan gum and said cationic cross-linking agent is calcium sulfate.

## Patentansprüche

1. Pharmazeutischer Trägerstoff zur verzögerten Freisetzung für orale Feststoffdosierungsformen, aufweisend:
• ein Heteropolysaccharid-Pflanzenharz, wobei der Prozentsatz des Heteropolysaccharid-Pflanzenharzes von 10 bis 40 Prozent des Trägerstoffes zur verzögerten Freisetzung beträgt,
• ein kationischer Vernetzer, der das Heteropolysaccharid-Pflanzenharz in Gegenwart wäßriger Lösungen zu vernetzen vermag, wobei das Verhältnis des Heteropolysaccharid-Pflanzenharzes zu dem kationischen Vernetzer von 1:1 bis 3,5:1 beträgt;
• ein indifferentes pharmazeutisches Verdünnungsmittel, das aus der Gruppe bestehend aus einem Monosaccharid, einem Disaccharid, einem mehrwertigen Alkohol, einer Zellulose, einer Stärke sowie deren Gemischen ausgewählt ist, wobei der Prozentsatz des indifferenten Verdünnungsmittels von 60 bis 85 Gewichtsprozent des Trägerstoffes zur verzögerten Freisetzung beträgt und wobei der Trägerstoff zur verzögerten Freisetzung in der Form einer trockenen Mischung oder eines Granulates vorliegt,
• wobei der pharmazeutische Trägerstoff zur verzögerten Freisetzung frei von jeglichem Homopolysaccharid-Pflanzenharz ist.

2. Trägerstoff zur verzögerten Freisetzung nach Anspruch 1, wobei das indifferente pharmazeutische Verdünnungsmittel aus der aus Laktose, Dextrose, Rohrzucker, Fruchtzucker, mikrokristalliner Zellulose, Xylit, Sorbit sowie deren Gemischen bestehenden Gruppe ausgewählt ist.

3. Trägerstoff zur verzögerten Freisetzung nach Anspruch 1, wobei das Verhältnis des indifferenten pharmazeutischen Verdünnungsmittels zu dem Heteropolysaccharid-Pflanzenharz von 6:1 bis 2:1 beträgt.

4. Trägerstoff zur verzögerten Freisetzung nach Anspruch 1, wobei der kationische Vernetzer von 1 bis 20 Gewichtsprozent des Trägerstoffes zur verzögerten Freisetzung ausmacht.

5. Trägerstoff zur verzögerten Freisetzung nach Anspruch 1, wobei der kationische Vernetzer aus der aus Kalziumsulfat, Natriumchlorid, Kaliumsulfat, Natriumcarbonat, Lithiumchlorid, Trikaliumphopsphat, Natriumborat, Kaliumbromid, Kaliumfluorid, Natriumbicarbonat, Kalziumchlorid, Magnesiumchlorid, Natriumzitrat, Natriumacetat, Kalziumlaktat, Magnesiumsulfat, Natriumfluorid sowie deren Gemischen bestehenden Gruppe ausgewählt ist.

6. Trägerstoff zur verzögerten Freisetzung nach Anspruch 1, wobei der kationische Vernetzer Kalziumsulfat ist.

7. Trägerstoff zur verzögerten Freisetzung nach Anspruch 1, wobei das Heteropolysaccharid-Pflanzenharz Xanthan-Pflanzenharz ist.

8. Trägerstoff zur verzögerten Freisetzung nach Anspruch 1, wobei der Trägerstoff die Form eines Granulates hat.

9. Trägerstoff zur verzögerten Freisetzung nach Anspruch 1, wobei das indifferente pharmazeutische Verdünnungsmittel in einer unmittelbar verdichtbaren Form vorliegt.

10. Trägerstoff zur verzögerten Freisetzung nach Anspruch 1, wobei das Heteropolysaccharid-Pflanzenharz, das indifferente pharmazeutische Verdünnungsmittel und der kationische Vernetzer mit einem hydrophoben Material granuliert sind, das aus der aus einer Alkylzellulose, einem Copolymer aus Acrylsäureestern und Methacrylsäureestern, Wachsen, Schellack, Maiskleber, hydrierten Pflanzenölen sowie jegliche Mischungen derselben bestehenden Gruppe ausgewählt ist, wobei das hydrophobe Material in einer Menge hinzugenommen ist, die eine Verlangsamung der Hydratisierung des einer Umgebungsflüssigkeit ausgesetzten Geliermittels bewirkt.

11. Trägerstoff zur verzögerten Freisetzung nach Anspruch 10, wobei das hydrophobe Material Ethylzellulose ist.

12. Ein als pharmazeutischer Trägerstoff zur verzögerten Freisetzung für orale Feststoffdosierungsformen verwendbares Granulat, aufweisend:
• ein Heteropolysaccharid-Pflanzenharz, wobei der Prozentsatz des Heteropolysaccharid-Pflanzenharzes von 10 bis 40 Prozent des Trägerstoffes zur verzögerten Freisetzung beträgt,
• ein kationischer Vernetzer, der das Heteropolysaccharid-Pflanzenharz in Gegenwart wäßriger Lösungen zu vernetzen vermag, wobei das Verhältnis des Heteropolysaccharid-Pflanzenharzes zu dem kationischen Vernetzer von 1:1 bis 3,5:1 beträgt;
• ein indifferentes pharmazeutisches Verdünnungsmittel, das aus der Gruppe bestehend aus einem Monosaccharid, einem Disaccharid, einem mehrwertigen Alkohol, einer Zellulose, einer Stärke sowie deren Gemischen ausgewählt ist, wobei der Prozentsatz des indifferenten Verdünnungsmittels von 60 bis 85 Gewichtsprozente vom Trägerstoff zur verzögerten Freisetzung beträgt;
• wobei das Heteropolysaccharid-Pflanzenharz, der kationische Vernetzer und das indifferente pharmazeutische Verdünnungsmittel durch ein Naßgranulationsverfahren zu körnigen Partikeln zusammengeballt sind,
• wobei der pharmazeutische Trägerstoff zur verzögerten Freisetzung frei von jeglichem Homopolysaccharid-Pflanzenharz ist.

13. Trägerstoff zur verzögerten Freisetzung nach Anspruch 12, wobei das indifferente pharmazeutische Verdünnungsmittel aus der aus Laktose, Dextrose, Rohrzucker, Fruchtzucker, mikrokristalliner Zellulose, Xylit, Sorbit sowie deren Gemischen bestehenden Gruppe ausgewählt ist.

14. Trägerstoff zur verzögerten Freisetzung nach Anspruch 12, wobei das Verhältnis des indifferenten pharmazeutischen Verdünnungsmittels zu dem Heteropolysaccharid-Pflanzenharz von 6:1 bis 2:1 beträgt.

15. Trägerstoff zur verzögerten Freisetzung nach Anspruch 12, wobei der kationische Vernetzer von 1 bis 20 Gewichtsprozent des Trägerstoffes zur verzögerten Freisetzung ausmacht.

16. Trägerstoff zur verzögerten Freisetzung nach Anspruch 12, wobei der kationische Vernetzer aus der aus Kalziumsulfat, Natriumchlorid, Kaliumsulfat, Natriumcarbonat, Lithiumchlorid, Trikaliumphopsphat, Natriumborat, Kaliumbromid, Kaliumfluorid, Natriumbicarbonat, Kalziumchlorid, Magnesiumchlorid, Natriumzitrat, Natriumacetat, Kalziumlaktat, Magnesiumsulfat, Natriumfluorid sowie deren Gemischen bestehenden Gruppe ausgewählt ist.

17. Trägerstoff zur verzögerten Freisetzung nach Anspruch 12, wobei der kationische Vernetzer Kalziumsulfat ist.

18. Trägerstoff zur verzögerten Freisetzung nach Anspruch 12, wobei das Heteropolysaccharid-Pflanzenharz Xanthan-Pflanzenharz ist.

19. Verfahren zum Zubereiten eines Trägerstoffes zur verzögerten Freisetzung nach einem der Patentansprüche 1 bis 11, aufweisend:
• Naßgranulieren eines Heteropolysaccharid-Pflanzenharzes, eines zum Vernetzen des Heteropolysaccharid-Pflanzenharzes in Gegenwart wäßriger Lösungen befähigten kationischen Vernetzers und eines indifferenten pharmazeutischen Verdünnungsmittels, das aus der Gruppe bestehend aus einem Monosaccharid, einem Disaccharid, einem mehrwertigen Alkohol, einer Zellulose, einer Stärke sowie deren Gemischen ausgewählt ist, und
• Trocknen des Gemisches, um einzelne Trägerstoffpartikel zu erhalten,
• wobei der pharmazeutische Trägerstoff zur verzögerten Freisetzung frei von jeglichem Homopolysaccharid-Pflanzenharz ist.

20. Verfahren nach Anspruch 19, wobei der Prozentsatz des Heteropolysaccharid-Pflanzenharzes von 10 bis 40 Prozent des Granulates beträgt, wobei der Prozentsatz des indifferenten Verdünnungsmittels von 60 bis 85 Gewichtsprozente des Granulates ausmacht, und wobei das Verhältnis des Heteropolysaccharid-Pflanzenharzes zu dem kationischen Vernetzer von 1:1 bis 3,5:1 beträgt.

21. Verfahren nach Anspruch 20, wobei das Heteropolysaccharid-Pflanzenharz Xanthan-Pflanzenharz und der kationische Vernetzer Kalziumsulfat ist.

## Revendications

1. Un excipient pharmaceutique à libération prolongée pour des formes dosées solides à administration orale, comprenant :
une gomme hétéropolysaccharide, le pourcentage de ladite gomme hétéropolysaccharide représentant de 10 à 40% de l'excipient à libération prolongée ;
un agent de réticulation cationique capable de se réticuler avec ladite gomme hétéropolysaccharide en présence de solutions aqueuses, la proportion de ladite gomme hétéropolysaccharide par rapport audit agent de réticulation cationique étant de 1:1 à 3,5:1 ;
un diluant pharmaceutique inerte choisi dans le groupe constitué par un monosaccharide, un disaccharide, un alcool polyhydrique, de la cellulose, de l'amidon, et leurs mélanges, le pourcentage dudit diluant inerte représentant de 60 à 85% en poids de l'excipient à libération prolongée et l'excipient à libération prolongée étant sous forme d'un mélange ou d'un granulat sec,
dans lequel ledit excipient pharmaceutique à libération prolongée est exempt de toute gomme homopolysaccharide.

2. L'excipient à libération prolongée de la revendication 1, dans lequel ledit diluant pharmaceutique inerte est choisi dans le groupe constitué par le lactose, le dextrose, le sucrose, le fructose, les celluloses microcristallines, le xylitol, le sorbitol et leurs mélanges.

3. L'excipient à libération prolongée de la revendication 1, dans lequel la proportion dudit diluant pharmaceutique inerte par rapport à ladite gomme hétéropolysaccharide est de 6:1 à 2:1.

4. L'excipient à libération prolongée de la revendication 1, dans lequel ledit agent de réticulation cationique représente de 1 à 20% en poids dudit excipient à libération prolongée.

5. L'excipient à libération prolongée de la revendication 1, dans lequel ledit agent de réticulation cationique est choisi dans le groupe constitué par le sulfate de calcium, le chlorure de sodium, le sulfate de potassium, le carbonate de sodium, le chlorure de lithium, le phosphate tripotassique, le borate de sodium, le bromure de potassium, le fluorure de potassium, le bicarbonate de sodium, le chlorure de calcium, le chlorure de magnésium, le citrate de sodium, l'acétate de sodium, le lactate de calcium, le sulfate de magnésium, le fluorure de sodium et leurs mélanges.

6. L'excipient à libération prolongée de la revendication 1, dans lequel ledit agent de réticulation cationique est le sulfate de calcium.

7. L'excipient à libération prolongée de la revendication 1, dans lequel ladite gomme hétéropolysaccharide est la gomme de xanthane.

8. L'excipient à libération prolongée de la revendication 1, dans lequel ledit excipient est sous forme d'un granulat.

9. L'excipient à libération prolongée de la revendication 1, dans lequel ledit diluant pharmaceutique inerte est sous une forme directement compressible.

10. L'excipient à libération prolongée de la revendication 1, dans lequel ladite gomme hétéropolysaccharide, ledit diluant pharmaceutique inerte et ledit agent de réticulation cationique sont granulés avec une matière hydrophobe choisie dans le groupe constitué par une alkylcellulose, un copolymère d'esters d'acides acryliques et métacryliques, des cires, de la shellaque, de la zéine, des huiles végétales hydrogénées et des mélanges de l'un quelconque des précédents, ladite matière hydrophobe étant incluse en quantité efficace pour ralentir l'hydratation dudit agent gélifiant quand il est exposé à un fluide environnemental.

11. L'excipient à libération prolongée de la revendication 10, dans lequel ladite matière hydrophobe est l'éthylcellulose.

12. Un granulat utilisable comme excipient pharmaceutique à libération prolongée pour formes dosées solides pour l'administration orale, comprenant :
une gomme hétéropolysaccharide, le pourcentage de ladite gomme hétéropolysaccharide représentant de 10 à 40% de l'excipient à libération prolongée ;
un agent de réticulation cationique capable de se réticuler avec ladite gomme hétéropolysaccharide en présence de solutions aqueuses, la proportion de ladite gomme hétéropolysaccharide par rapport audit agent de réticulation cationique étant de 1:1 à 3,5:1 ;
un diluant pharmaceutique inerte choisi dans le groupe constitué par un monosaccharide, un disaccharide, un alcool polyhydrique, de la cellulose, de l'amidon, et leurs mélanges, le pourcentage dudit diluant inerte représentant de 60 à 85% en poids de l'excipient à libération prolongée ;
ladite gomme hétéropolysaccharide, ledit agent de réticulation cationique et ledit diluant pharmaceutique inerte ayant été aggloméré en particules granulaires par une méthode de granulation à l'humidité ;
dans lequel l'excipient pharmaceutique à libération prolongée est exempt de toute gomme homopolysaccharide.

13. Le granulat de la revendication 12, dans lequel ledit diluant pharmaceutique inerte est choisi dans le groupe constitué par le lactose, le dextrose, le sucrose, le fructose, les celluloses microcristallines, le xylitol, le sorbitol et leurs mélanges.

14. Le granulat de la revendication 12, dans lequel la proportion dudit diluant pharmaceutique inerte par rapport à ladite gomme hétéropolysaccharide est de 6:1 à 2:1.

15. Le granulat de la revendication 12, dans lequel ledit agent de réticulation cationique représente de 1 à 20% en poids dudit excipient à libération prolongée.

16. Le granulat de la revendication 12, dans lequel ledit agent de réticulation cationique est choisi dans le groupe constitué par le sulfate de calcium, le chlorure de sodium, le sulfate de potassium, le carbonate de sodium, le chlorure de lithium, le phosphate tripotassique, le borate de sodium, le bromure de potassium, le fluorure de potassium, le bicarbonate de sodium, le chlorure de calcium, le chlorure de magnésium, le citrate de sodium, l'acétate de sodium, le lactate de calcium, le sulfate de magnésium, le fluorure de sodium et leurs mélanges.

17. Le granulat de la revendication 12, dans lequel ledit agent de réticulation cationique est le sulfate de calcium.

18. Le granulat de la revendication 12, dans lequel ladite gomme hétéropolysaccharide est la gomme de xanthane.

19. Une méthode pour la préparation d'un excipient à libération prolongée selon l'une quelconque des revendications 1-11, comprenant les étapes consistant à:
granuler à l'humidité une gomme hétéropolysaccharide, un agent de réticulation cationique capable de se réticuler avec ladite gomme hétéropolysaccharide en présence de solutions aqueuses et un diluant pharmaceutique inerte choisi dans le groupe constitué par un monosaccharide, un disaccharide, un alcool polyhydrique, une cellulose, un amidon et leurs mélanges, et
sécher le mélange de manière à obtenir des particules distinctes d'excipient,
dans laquelle l'excipient à libération prolongée est exempt de toute gomme homopolysaccharide.

20. La méthode de la revendication 19, dans laquelle le pourcentage de ladite gomme hétéropolysaccharide représente de 10 à 40% en poids du granulat, le pourcentage dudit diluant inerte représente 60 à 85% en poids de la granulation et la proportion de ladite gomme hétéropolysaccharide par rapport audit agent de réticulation cationique est de 1:1 à 3,5:1.

21. La méthode de la revendication 20, dans laquelle ladite gomme hétéropolysaccharide est la gomme de xanthane et ledit agent de réticulation cationique est le sulfate de calcium.
